# FASCICULE DE BREVET EUROPEEN

(11) **EP 3 331 579 B1**
(45) Date de publication et mention de la délivrance du brevet: **11.03.2020**
(21) Numéro de dépôt: 16750805.0
(22) Date de dépôt: 08.08.2016
(51) Int. Cl.: C23C 18/18, C22C 14/00, C08J 7/12, C08F 2/48, C08F 12/30, B32B 27/30, B32B 15/082, A61L 27/34, A61L 27/04, C08J 7/04

(54) **PROCEDE DE GREFFAGE DE POLYMERES BIOACTIFS SUR DES MATERIAUX METALLIQUES**
VERFAHREN ZUM PFROPFEN VON BIOAKTIVEN POLYMEREN AUF METALLISCHE WERKSTOFFE
PROCESS FOR GRAFTING BIOACTIVE POLYMERS ONTO METALLIC MATERIALS

(30) Priorité: 07.08.2015 FR 1557621
(43) Date de publication de la demande: 13.06.2018
(73) Titulaire: Univ Paris XIII Paris-Nord Villetaneuse, 93430 Villetaneuse (FR); Centre National de la Recherche Scientifique (CNRS), 75016 Paris (FR)
(72) Inventeur: MIGONNEY, Véronique, 95600 Eaubonne (FR); BAUMANN, Jean-Sébastien, 93800 Epinay Sur Seine (FR); FALENTIN-DAUDRE, Céline, 95170 Deuil La Barre (FR); CHOUIRFA, Hamza, 30540 Milhaud (FR)
(74) Mandataire: Regimbeau
(86) Numéro de dépôt international: PCT/EP2016/068909
(87) Numéro de publication internationale: WO 2017/025519

(56) Documents cités:
- WO-A1-96/20023
- WO-A1-2010/125190
- WO-A2-2007/141460
- Z.-Y. QIU ET AL: "Advances in the surface modification techniques of bone-related implants for last 10 years", REGENERATIVE BIOMATERIALS, vol. 1, no. 1, 1 novembre 2014 (2014-11-01), pages 67-79, XP055267449, ISSN: 2056-3418, DOI: 10.1093/rb/rbu007

## Description

### DOMAINE DE L'INVENTION

La présente invention concerne un procédé de greffage de polymères sur un matériau métallique, les matériaux métalliques greffés susceptibles d'être obtenus par ce procédé et des applications de ces derniers. Plus spécifiquement, l'invention concerne un procédé de greffage direct de polymères à la surface de matériaux métalliques.

### ARRIERE-PLAN TECHNOLOGIQUE

L'implantation d'une prothèse articulaire ou dentaire dans un site osseux engendre une cascade de réactions tissus/implants intitulée « réponse de l'hôte » qui, si elle est contrôlée, permet d'aboutir *in fine* à « l'ostéointégration de l'implant », processus menant notamment à l'intégration de la prothèse dans le tissu osseux grâce à un contact intime tissu osseux/prothèse et à une minimisation de l'épaisseur du tissus fibreux à l'interface. Dans le cas contraire, ces réactions peuvent conduire à une nouvelle intervention chirurgicale, voire au retrait de la prothèse.
En effet, le matériau implanté est considéré comme un « corps étranger » par le système vivant, ce qui déclenche une réponse inflammatoire, pouvant conduire à une encapsulation fibreuse, et ainsi, au rejet de l'implant. De plus, lorsqu'un tissu « fibro-inflammatoire » est produit autour de l'implant (tissu qui n'a ni les activités biologiques, ni les caractéristiques mécaniques du tissu osseux), la probabilité d'un descellement aseptique (désolidarisation de l'implant) ou d'une infection du site de l'implantation s'en trouve accrue.
Le matériau prothétique apparaît en effet comme un support privilégié pour l'adhésion et la colonisation par des bactéries (éventuellement par la formation d'un « biofilm » bactérien résistant notamment aux antibiotiques), étape initiatrice d'une infection pouvant conduire à une septicémie, une endocardite ou une ostéomyélite, notamment dans le cas d'infections aux staphylocoques.
Le processus d'ostéointégration peut ainsi être affecté par la technique chirurgicale et la présence de germes lors de l'implantation, mais aussi pour une grande partie par les propriétés physicochimiques du matériau, telles que la topographie de surface, sa rugosité, ou sa composition chimique.
Pour améliorer l'ostéointégration des implants, notamment métalliques, les revêtements « hydroxyapatite » sont encore aujourd'hui utilisés, car ils permettent un bon ancrage osseux tout en offrant de bonnes performances mécaniques dans un temps relativement court.
Par ailleurs, des matériaux prothétiques prometteurs, métalliques ou polymériques, dont la surface est modifiée par greffage de « polymères bioactifs », destinés à améliorer la biointégration (notamment par diminution de la réponse inflammatoire), ont été développés.
Plusieurs procédés ont été décrits pour la préparation de ces matériaux, procédés que l'on peut classer en deux catégories : les procédés par greffage « indirect » du polymère bioactif, et les procédés par greffage « direct ».
Au sens de la présente invention, un « procédé de greffage indirect » inclut une première étape de fonctionnalisation de la surface du matériau métallique à l'aide d'une molécule de faible poids moléculaire, qui sert de point d'ancrage pour greffer le polymère bioactif, le polymère étant soit préformé, soit formé *in situ* par polymérisation dans une étape subséquente. A titre d'exemple de greffage indirect, on peut citer notamment le procédé décrit dans l'article de Li et al. (Langmuir 2011, 27(8), 4848-4856). Le procédé divulgué dans ce document fait intervenir une première étape de greffage par réaction radicalaire d'un composé oléfinique (de préférence le TMG-C10) avec une initiation par irradiation UV d'une durée supérieure à 2h30. Toutefois, ces composés ne subissent pas de réaction de polymérisation. Ils sont bifonctionnels, et leur second groupe fonctionnel sert de point d'ancrage pour greffer le polymère à proprement parler. Ainsi, le composé oléfinique (ou coupleur), une fois greffé, sert de point d'ancrage dans un procédé de greffage « indirect », où le polymère en tant que tel est introduit par la suite *via* le second groupe fonctionnel.
Par opposition, un « procédé de greffage direct » s'entend, au sens de la présente invention, d'un procédé dans lequel le polymère est greffé directement à la surface du matériau métallique, sans molécule intermédiaire servant de point d'ancrage pour se lier au polymère. En d'autres termes, dans un matériau obtenu par un procédé de greffage direct, il n'existe aucune molécule intermédiaire entre la surface du matériau et le polymère bioactif greffé. Un tel procédé est notamment plus économique, plus rapide et plus efficace qu'un procédé de greffage indirect. A titre d'exemple, on peut citer le procédé décrit dans WO 2007/141460. Celui-ci comprend les étapes suivantes :
- des espèces actives donneurs de radicaux libres sont générées à la surface du matériau prothétique ; et
- le matériau prothétique sur lequel des espèces actives ont été générées est mis en présence d'au moins un monomère porteur d'une fonction permettant une polymérisation radicalaire, sa polymérisation radicalaire permettant, en l'absence d'oxygène, la formation d'un polymère bioactif.
Un tel procédé permet notamment de produire des matériaux prothétiques comprenant un taux de greffage particulièrement élevé, compris entre 3 et 10 µg.cm⁻². Toutefois, le procédé de WO 2007/141460, qui fait appel à une étape d'amorçage thermique de la polymérisation radicalaire, est particulièrement long : temps total de mise en oeuvre d'environ 14-15h (voir notamment l'exemple 1.4 de WO 2007/141460).
Il existe donc un besoin pour un procédé permettant d'obtenir des matériaux métalliques avec un taux de greffage de polymères élevé (notamment supérieur à 1,5 µg.cm⁻²) en un temps raisonnable, par exemple inférieur à 2h30.

### RESUME DE L'INVENTION

Les Inventeurs ont ainsi mis au point un procédé de greffage de polymères sur un matériau métallique conduisant à des taux de greffage élevés, tout en diminuant de manière spectaculaire (facteur supérieur à 10) la durée globale de mise en oeuvre du procédé selon WO 2007/141460.
Plus particulièrement, les inventeurs ont réduit la durée nécessaire pour effectuer l'étape de polymérisation radicalaire en ayant recours à une étape d'amorçage par irradiation UV, notamment avec une gamme de puissance d'irradiation spécifique. L'invention concerne un procédé de greffage comme décrit dans les revendications.

### DEFINITIONS

Sauf indication contraire, dans la présente description, les indices m, n, p, q, sont des nombres entiers.
Par « matériau métallique », on entend au sens de la présente invention un matériau essentiellement constitué de métal ou un matériau dont la surface est essentiellement constituée de métal. Lorsque le matériau n'est pas essentiellement constitué de métal, l'épaisseur de la surface essentiellement constituée de métal est de préférence supérieure à 1 µm, de façon plus préférée supérieur à 10µm.
Par « alliage », on entend au sens de la présente invention un matériau constitué d'une combinaison d'un élément métallique avec un ou plusieurs autres éléments chimiques. De préférence tous les éléments chimiques constituants l'alliage sont des métaux. Par exemple, l'alliage de titane selon l'invention peut être une combinaison de titane avec du vanadium et de l'aluminium (e.g. TA6V4AI comprenant 6% de Vanadium et 4 % d'aluminium). Un alliage peut comprendre un métal en combinaison avec élément chimique représentant jusqu'à 45 % en masse de l'alliage. Par exemple, un alliage Ti-25Nb-3Fe comprend jusqu'à 25 % de Niobium et 3 % de Fer et un alliage Ti-24.8Nb-40.7Zr comprend jusqu'à 24,8 % de Niobium et 40,7 % de Zirconium. Un alliage peut être la combinaison de nombreux éléments métalliques comme par exemple le Ti-10,1Ta-1,7Nb-1,6Zr qui est une combinaison de 4 éléments différents.

Par « matériau essentiellement constitué de X », on entend au sens de la présente invention un matériau comprenant uniquement X, X pouvant être un élément métallique ou un alliage, et éventuellement des traces d'autres constituants. Ainsi, un matériau essentiellement constitué de X comprend au moins 95% en poids, de préférence au moins 97% en poids de X, éventuellement en combinaison avec l'oxygène, par rapport au poids total du matériau. En effet, il est entendu qu'un matériau métallique peut comprendre une couche d'oxydation native en surface. Ainsi, un matériau métallique essentiellement constitué de X comprend au plus 5%, de préférence au plus 3%, en poids d'un élément différent de l'oxygène et de X, par rapport au poids total du matériau.

Par « matériau prothétique », on entend au sens de la présente invention un matériau utilisable dans la fabrication d'un implant médical tel qu'une prothèse, en particulier une prothèse de hanche ou un implant dentaire. De préférence, le matériau prothétique selon l'invention est essentiellement constitué de métal.

Par « composé pur », on entend au sens de la présente invention que le composé présente une pureté de 100%. Par « composé essentiellement pur » on entend au sens de la présente invention que le composé est utilisé tel quel, sans être mélangé à un autre composé, et en particulier il n'est pas mis en solution. Un composé « essentiellement pur » peut néanmoins contenir jusqu'à 5% en poids (de préférence moins de 3%) d'impuretés par rapport au poids total du composé. Ainsi, un monomère tel que l'acide acrylique de grade technique commercial est un composé essentiellement pur.

Par « polymère bioactif », on entend au sens de la présente invention un polymère permettant d'améliorer l'ostéointégration du matériau métallique, de préférence prothétique, sur lequel il est greffé. De préférence, un polymère bioactif est en particulier capable d'orienter les réponses cellulaires eucaryotes et/ou procaryotes au site d'intégration de l'implant prothétique fabriqué à partir d'un matériau métallique susceptible d'être obtenu selon le procédé de la présente invention, et de prévenir le développement d'une infection. Un tel polymère comporte de préférence des groupements ioniques. De façon plus préférée, le polymère bioactif comprend des groupements phosphonates, carboxylates et/ou sulfonates.
L'angle de contact d'un matériau se mesure selon des méthodes bien connues de l'homme du métier. Par exemple, on peut utiliser une méthode de mesure de l'angle de contact à partir d'une goutte d'eau déposée à la surface du matériau (matériau métallique oxydé ou matériau métallique greffé) via l'appareil KRUSS : DSA100 qui permet de connaître les modifications du caractère hydrophile ou hydrophobe de la surface.
Au sens de la présente invention, une oléfine est un monomère comprenant au moins une insaturation carbone - carbone (double liaison carbone-carbone C=C ou triple liaison C≡C) non aromatique, de préférence une double liaison. Par monomères présentant au moins une insaturation carbone - carbone non aromatique, on entend selon l'invention des monomères présentant une ou deux, de préférence une, double ou triple liaison, avantageusement une double liaison, et plus particulièrement le motif CH₂=CH-.
Dans la présente description, l'acronyme « UV » est synonyme d'ultraviolet. Ainsi, lorsque l'on parle d'« irradiation UV », on entend « irradiation aux rayons ultraviolets ». Par « polymérisation radicalaire », on entend une polymérisation en chaîne faisant intervenir comme espèce active des radicaux. Elle comprend une étape d'amorçage, de propagation, de terminaison, et éventuellement de transfert de chaîne.
L'étape d'amorçage consiste en la formation d'un radical issu de l'amorceur sur la première unité monomère (dans cet exemple, un monomère oléfinique) :

X˙ + CH₂=CHR → X-CH₂-HC˙R.

Ladite étape d'amorçage peut être effectuée avantageusement sans chauffage thermique additionnel à l'irradiation UV.
La propagation est la principale étape de la polymérisation radicalaire. C'est au cours de cette étape que la chaîne polymérique se forme à partir de la surface métallique par additions successives d'unités monomères sur la chaine en croissance.

Le nombre d'occurrences de la réaction de propagation gouverne le degré de polymérisation en nombre de la chaîne formée et donc la masse molaire du polymère formé.

X-(CH₂-CHR)*n-*CH₂-HC^{*}R + CH₂=CHR → X⁻(CH₂⁻CHR)_{*n*+1}-CH₂⁻HC^{*}R.

Les réactions de terminaison sont de plusieurs types. La terminaison peut résulter de l'addition sur la chaine en croissance d'une molécule d'amorceur, de solvant, d'une impureté présente dans le milieu etc... D'autres réactions de terminaison, la recombinaison et la dismutation, mettent en jeu deux chaines en croissance. Dans une réaction de recombinaison, deux chaines reforment une liaison covalente :

X-(CH₂-CHR)*ₙ*-CH₂-HC^{*}R + R^{*}CH-CH₂-(CHR-CH₂)*ₘ*-X → X-(CH₂-CHR)*ₙ*-CH₂-HRC-CHR-CH₂-(CHR-CH₂)*ₘ*-X

Dans une réaction de dismutation, deux chaines donnent lieu à une réaction de transfert d'hydrogène, suivie d'une recombinaison. Le résultat global peut s'écrire :

X-(CH₂-CHR)*ₙ*-CH₂-HC^{*}R + R^{*}CH-CH₂-(CHR-CH₂)*ₘ*-X → X-(CH₂-CHR)*ₙ*-CH₂-CH₂R + CRH=CH-(CHR-CH₂)*ₘ*-X.

La proportion relative de ces modes de terminaison dépend essentiellement du type de monomère employé, de l'accessibilité des sites radicalaires c'est-à-dire de l'encombrement stérique des sites actifs.
Le taux de greffage du polymère (de préférence bioactif) du matériau métallique greffé est exprimé en µg.cm⁻². Il est mesuré par exemple à l'aide d'un dosage colorimétrique au bleu de toluidine, lorsque le polymère comprend des groupes sulfonates, carboxylates ou phosphonates.
Au sens de la présente invention, on entend par « acide » un acide organique ou minéral dont le pKa dans l'eau est inférieur à 3.
Par « alkyle en C₁-C₁₀» on entend au sens de la présente invention une chaîne hydrocarbonée saturée de 1 à 10 atomes de carbone, linéaire ou ramifiée. A titre d'exemple d'alkyle en C₁-C₁₀, on peut citer notamment le méthyle, l'éthyle, le propyle, le n-butyle, le s-butyle, le tert-butyle, le pentyle, l'isopentyle, le n-hexyle. De préférence, l'alkyle en C₁-C₁₀ est un alkyle en C₁-C₄. Le méthyle et l'éthyle sont particulièrement préférés.
Par « hétéroaryle », on entend, au sens de la présente invention, un groupe aromatique comprenant 5 à 10 atomes cycliques dont un ou plusieurs hétéroatomes, avantageusement 1 à 4 et encore plus avantageusement 1 ou 2, tels que par exemple des atomes de soufre, azote ou oxygène, les autres atomes cycliques étant des atomes de carbone. Des exemples de groupes hétéroaryle sont les groupes furyle, thiényle, pyrrolyle, pyridinyle, pyrimidinyle, pyrazolyle, imidazolyle, triazolyle, tétrazolyle ou encore indyle.
Au sens de la présente invention, on entend par « oxydation contrôlée », une étape d'oxydation permettant de générer spécifiquement des fonctions hydroperoxydes - OOH à la surface du matériau métallique. Ainsi, l'oxydation contrôlée selon l'invention permet d'obtenir un résultat différent de l'oxydation non contrôlée que pourrait subir le matériau métallique conduisant à une couche d'oxydation naturelle, qui est essentiellement constituée de fonctions OH dont la répartition et la densité ne sont pas homogènes sur la surface du matériau métallique.
Au sens de la présente invention, on entend par « absence d'O₂ », un taux de O₂ inférieur à 1 % en volume, de façon préférée inférieur à 0,5% en volume par rapport au volume total du gaz considéré.
Au sens de la présente invention, on entend par « puissance surfacique », la puissance de l'irradiation UV à la surface du matériau métallique. Elle est notamment dépendante de la puissance de la lampe utilisée et de la distance entre la lampe et l'échantillon. Elle peut être aisément mesurée par l'homme du métier, par exemple à l'aide d'un radiomètre à microprocesseur (e.g. VLX-3W, VILBER LOURMAT). Aux fins de la mesure, on considère la puissance stabilisée, par exemple lorsque la lampe est allumée depuis 1 heure ou plus.

### DESCRIPTION DETAILLEE DE L'INVENTION

### Procédé de greffage

Ainsi, l'invention a pour objet un procédé de greffage de polymères sur un matériau métallique, comprenant les étapes suivantes :
a) l'oxydation de la surface du matériau métallique, conduisant à un matériau métallique oxydé, et
b) le greffage d'un polymère à la surface dudit matériau métallique oxydé par polymérisation radicalaire d'un monomère, ladite polymérisation radicalaire comprenant une étape d'amorçage et une étape de propagation,
ladite étape d'amorçage étant effectuée par irradiation UV avec une source UV diffusant une puissance à la surface du matériau supérieure à 72 mW, de préférence comprise entre 72 mW.cm⁻² et 20 W.cm⁻², de manière préférée entre 72 mW.cm⁻² et 226 mW.cm⁻², de manière encore préférée entre 91 mW.cm⁻² et 226 mW.cm⁻², de manière préférée entre toutes égale à environ 162 mW.cm⁻²,
ladite irradiation UV étant réalisée pendant une durée supérieure à 30 minutes et inférieure à 180 minutes, de préférence inférieure ou égale à 120 minutes, de manière encore préférée inférieure ou égale à 90 minutes,
ledit procédé conduisant à un matériau métallique greffé.
Le procédé selon l'invention est un procédé de greffage direct. Ainsi, le procédé de greffage de la présente invention est exempt de toute étape de greffage d'une molécule intermédiaire ou coupleur entre le matériau métallique et le polymère greffé.

### Matériau métallique

Le matériau métallique selon l'invention est de préférence un matériau prothétique en titane ou en alliage de titane.
On peut citer comme exemple de matériaux métalliques, de préférence prothétiques, les matériaux à base de titane, d'aluminium, d'acier, de chrome, de cobalt, de niobium, de tantale, de vanadium, d'iridium, de zirconium, d'or et leurs alliages.
Les matériaux métalliques comprennent généralement en surface une couche d'oxydation native qui se forme spontanément en présence d'air, comprenant ou essentiellement constituée de groupes M-OH dont la densité et la répartition est aléatoire, M représentant un atome d'un des métaux de l'alliage, notamment un atome d'un métal cité parmi les listes ci-dessus.
Le matériau métallique (de préférence prothétique) est en titane ou en alliage de titane. En effet, le titane est connu pour ses propriétés de biocompatibilité. Typiquement, un matériau en titane est essentiellement constitué de titane, et/ou d'oxyde de titane. Dans tous les cas, le matériau en titane comprend en surface une couche d'oxydation native qui se forme spontanément en présence d'air, comprenant des groupes Ti-OH dont la densité et la répartition est aléatoire.
Avantageusement, l'alliage de titane est à base de nickel, de vanadium, d'aluminium niobium et/ou de molybdène, de préférence c'est un alliage de titane, d'aluminium et de vanadium, de manière préférée entre toutes il s'agit du TiAl₆V₄.

### Etape optionnelle de polissage

Le procédé selon l'invention peut comprendre une étape préalable de polissage.
Cette étape est effectuée de préférence préalablement à l'étape de décapage qui est décrite ci-après.
Ainsi, le matériau métallique peut être poli par abrasion pour limiter la rugosité de la surface du matériau.
Dans un mode particulier de réalisation de l'invention, les matériaux métalliques sont polis avec du papier abrasif et de préférence avec différents papiers abrasifs de granulométries décroissantes. Plus spécifiquement, le polissage mécanique des matériaux métalliques peut être réalisé à l'aide d'un bras automatique monté sur une polisseuse rotative, avec du papier abrasif de granulométries décroissantes. Ainsi, on peut successivement utiliser des papiers abrasifs de grades 500 puis 1200.
Avantageusement, le matériau métallique est également lavé, de préférence successivement au polissage, notamment par trempage dans une solution d'acétone puis avec de l'eau, puis est de préférence séché.
De préférence toutefois, le procédé selon l'invention est dépourvu de toute étape de polissage.

### Rugosité du matériau métallique

Avantageusement, le matériau métallique utilisé présente une rugosité supérieure à 0,2 µm, de préférence supérieure à 1 µm, et façon plus préférée supérieure à 3 µm. Typiquement la rugosité est inférieure à 20 µm.
La rugosité est de préférence mesurée par microscopie à force atomique.
Si le matériau métallique de départ présente une rugosité trop élevée, alors le procédé comprend une étape de polissage telle que décrite ci-dessus pour obtenir une rugosité adaptée, notamment comprise entre 0.2 µm et 20 µm, de préférence comprise entre 3 µm et 10 µm.

### Etape de décapage

Avantageusement, le procédé selon l'invention comprend une étape de décapage, réalisée préalablement à l'étape d'oxydation, afin d'en améliorer l'efficacité. Lorsque le procédé selon l'invention comprend également une étape de polissage, l'étape de décapage est alors conduite entre l'étape de polissage et l'étape a) d'oxydation.
Le décapage est avantageusement un décapage chimique.

Un décapage chimique comprend typiquement une mise en contact du matériau métallique dans une solution aqueuse acide. Par exemple, le matériau métallique est trempé pendant une durée t_{decap} dans une solution aqueuse acide.
L'acide présente de préférence un pKa compris entre -2 et 3.
De préférence, la durée t_{decap} est comprise entre 0,5 et 10 minutes, de manière encore préférée entre 0,5 et 2 minutes.
La solution aqueuse acide utilisée pour l'étape de décapage est de préférence une solution aqueuse d'acide nitrique (HNO₃) ou une solution aqueuse comprenant un mélange d'acides fluorhydrique et nitrique (mélange HF/HNO₃), de préférence en des proportions volumiques HF/HNO₃ comprises entre 1:20 et 20:1.
Avantageusement, le pH de la solution aqueuse acide est compris entre -1 et 3, de manière encore préférée entre -1 et 1.
Ainsi, avantageusement, le décapage chimique comprend le trempage du matériau métallique dans une solution aqueuse acide de pH compris entre -1 et 1 pendant une durée t_{decap} comprise entre 0,5 et 1 minute, la solution aqueuse acide étant de préférence une solution aqueuse comprenant le mélange HF/HNO₃.
Des exemples de mise en oeuvre de cette étape de décapage pour des matériaux en titane ou alliage de titane sont notamment décrits par Liu et al (Materials Science and Engineering. R47 (2004) 49-121) et Takeuchi et al (Biomaterials 24 (2003) 1821-1827). Lorsque le procédé selon l'invention comprend également une étape de décapage préalablement à l'étape d'oxydation, l'étape d'oxydation est réalisée de préférence rapidement après le décapage. Ainsi, le temps entre la fin de l'étape de décapage et le début de l'étape d'oxydation est avantageusement inférieur à 16 heures, de préférence inférieur à 12 heures, de manière encore préférée inférieur à 6 heures, de façon encore plus préférée inférieur à 3 heures.
Avantageusement, l'étape de décapage comprend également le lavage (ou rinçage) du matériau décapé notamment avec de l'eau, en particulier de l'eau distillée.

### Oxydation (étape a)

De manière générale, l'oxydation peut être réalisée par tous les moyens d'oxydation de matériaux métalliques bien connus de l'homme du métier tels que l'oxydation par traitement avec une solution de peroxyde d'hydrogène, ou au moyen d'une forte température, ou avec une combinaison des deux techniques (Takemoto et al., 2004), l'oxydation anodique du titane dans une solution d'acide acétique ou dans un mélange d'électrolytes contenant des ions magnésium (Sul et al., 2005), et l'oxydation micro-arc sur des prothèses de titane (Li et al., 2004).
L'étape a) d'oxydation permet avantageusement de fournir une couche oxydée comprenant des groupes hydroperoxydes -OOH, de manière contrôlée, à la surface du matériau métallique.
En effet, les matériaux métalliques, notamment en titane ou en alliage de titane, présentent une couche d'oxydation native, dont la teneur en fonction M-OH n'est pas contrôlée (M représentant un atome métallique). Pour ces matériaux métalliques, l'étape d'oxydation permet notamment de former en surface des hydroperoxydes de métal (M-OOH) dont la densité peut être contrôlée.
Il en résulte que l'étape a) d'oxydation a une influence directe sur le taux de greffage du polymère, qui est très élevé, typiquement compris entre 1,5 et 10 µg.cm⁻². Ainsi, si l'étape a) du procédé selon l'invention est omise, le taux de greffage s'en trouve fortement diminué, notamment dans le cas des matériaux métalliques, tels que les matériaux, de préférence prothétiques, en titane, aluminium ou en alliages.
En outre, il semble que la couche d'oxydation permet d'éviter la migration d'ions métalliques toxiques, notamment lorsque le procédé est mis en oeuvre avec un alliage de titane de type TiAl₆V₄ (dans ce cas particulier les ions métalliques toxiques sont les ions vanadium et aluminium).
Dans un premier mode de réalisation, l'étape a) d'oxydation est réalisée par traitement chimique : ce mode de réalisation sera dans la suite appelé « oxydation par voie chimique ». L'oxydation par voie chimique est notamment une oxydation par mise en contact du matériau métallique avec une solution oxydante, notamment H₂O₂, ou une oxydation par ozonation. L'oxydation par voie chimique est en particulier réalisée par traitement du matériau avec une solution aqueuse comprenant un oxydant et un acide, de préférence un mélange H₂SO₄ et H₂O₂.
La mise en contact du matériau métallique avec la solution oxydante (notamment une solution comprenant H₂O₂) peut être réalisée par exemple, en versant la solution oxydante dans un récipient contenant le matériau métallique, ou le matériau métallique peut être plongé dans un récipient contenant la solution oxydante.
Dans le cas d'une oxydation par voie chimique par mise en contact du matériau métallique avec une solution oxydante, ladite solution oxydante est notamment un mélange acide /oxydant, en particulier H₂SO₄/H₂O₂. L'acide est de préférence un acide de pKa (dans l'eau) inférieur à 3, de manière encore préférée de pKa (dans l'eau) inférieur à 2. Avantageusement, l'acide est en solution aqueuse et choisi parmi l'acide fluorhydrique (HF), l'acide chlorhydrique (HCI), l'acide sulfurique (H₂SO₄) et leurs mélanges, de manière encore préférée il s'agit de H₂SO₄.
L'oxydant est de préférence H₂O₂. Néanmoins il peut être remplacé par l'ozone dans le cas d'une ozonation.
Ainsi, de préférence, dans ce mode de réalisation par traitement chimique, l'étape a) d'oxydation est réalisée avec un mélange aqueux H₂SO₄/H₂O₂.
La proportion d'acide par rapport à H₂O₂ peut varier dans une large mesure et l'homme du métier est à même de définir le rapport le plus efficace pour aboutir à un taux de greffage souhaité. De préférence, une solution H₂SO₄/H₂O₂ 50/50 (v/v) est utilisée pour oxyder le matériau métallique. La température utilisée est généralement la température ambiante (20-30°C), voire une température plus faible (par exemple, entre 0 et 20°C), la réaction d'oxydation pouvant être exothermique.
Par « mélange acide/H₂O₂ » on entend le mélange simultané ou séquentiel de la solution d'acide et de la solution de H₂O₂. Ainsi, soit les deux solutions sont mises en contact simultanément avec le matériau métallique à oxyder, soit le matériau métallique est d'abord mis en contact avec la solution d'acide, puis la solution de H₂O₂ est mise en contact dans un deuxième temps avec le matériau métallique.
Dans tous les cas, l'homme du métier pourra adapter le temps de contact du matériau métallique avec la solution oxydante en fonction de sa nature (titane pur ou sous forme d'alliage...), du mode d'oxydation chimique mis en oeuvre et du taux de greffage final souhaité.
Dans le cas d'un mélange acide/H₂O₂ simultané, le temps d'oxydation est de préférence de 1 à 10 minutes, de manière plus préférée de 3 à 6 minutes et de manière la plus préférée le temps d'oxydation est de 4 minutes. De préférence, ce temps d'oxydation s'applique à l'oxydation du titane ou d'un de ses alliages avec une solution H₂SO₄/H₂O₂.
Dans le cas de l'utilisation d'un mélange séquentiel, le matériau métallique peut être, par exemple, plongé dans la solution d'acide pendant au moins 10 secondes, de préférence pendant au moins 20 secondes, de préférence pendant au moins 30 secondes, de manière plus préférée pendant plus de 50 secondes, de manière préférée pendant plus de 1 minute, de manière préférée pendant plus de 2 minutes, de manière préférée pendant plus de 3 minutes, de manière préférée pendant plus de 4 minutes. Ce temps de mise en présence du titane, ou d'un de ses alliages, avec H₂SO₄ peut être largement supérieur et peut, par exemple, atteindre 30 minutes ou plus. Cependant, dans un mode préféré de réalisation, le temps de mise en présence du matériau métallique avec la solution d'acide est inférieur à 5 minutes, temps au-delà duquel une diminution du taux de greffage est observée. Cette procédure est en particulier appliquée dans le cas de l'oxydation du titane ou d'un de ses alliages, avantageusement mis en présence d'une solution de H₂SO₄.
De la même manière, le temps de mise en présence du matériau métallique avec la solution de H₂O₂ peut varier. De préférence, le matériau métallique, de préférence le titane ou un de ses alliages, est mis en présence de H₂O₂ pendant au moins 10 secondes, de préférence au moins 20 secondes, de préférence au moins 30 secondes, de préférence au moins 40 secondes, de préférence au moins 50 secondes, de préférence au moins 1 minute, de préférence au moins 2 minutes, de préférence pendant 2 à 3 minutes, et de manière la plus préférée pendant deux minutes après ajout de la solution de H₂O₂. Dans un mode préféré de réalisation, on préfère une action de H₂SO₄ sur le titane, ou un de ses alliages, pendant 1 minute puis une action de H₂O₂ sur le titane pendant 3 minutes.
L'oxydation chimique par trempage du matériau métallique dans une solution oxydante, notamment un mélange acide/H₂O₂ et de préférence un mélange H₂SO₄/H₂O₂, typiquement en des proportions 50/50 (v/v), est particulièrement préférée. Dans un mode de réalisation préféré, l'étape a) d'oxydation est réalisée par traitement chimique avec un mélange H₂SO₄ et H₂O₂, typiquement en des proportions 50/50 (v/v). Dans un autre mode de réalisation, l'étape a) d'oxydation est réalisée par traitement anodique (ou anodisation) ou par ozonation.
Typiquement, lors d'une oxydation par traitement anodique, le matériau métallique est mis en contact avec une solution électrolytique acide contenue dans une cuve électrochimique comprenant une électrode, puis subit une étape de mise sous tension électrochimique, le matériau métallique jouant le rôle de l'anode dans la cuve électrochimique. A l'anode (matériau métallique), se produit une réaction d'oxydation des protons selon l'équation suivante : 2e⁻ + 2H⁺ → H₂.
Avantageusement, le potentiel appliqué à l'anode est compris entre 10 et 250 V vs électrode normale à l'hydrogène (NHE), de préférence entre 20 et 200 V vs NHE.
En outre, avantageusement, l'intensité appliquée à l'anode est comprise entre 0.1 et 50 mA/cm², de préférence entre 1 et 50 mA/cm².
De préférence, la solution électrolytique comprend un acide possédant au moins un pKa inférieur à 2.5, par exemple un acide possédant au moins un pKa compris entre - 10 et 2.5. Par exemple, on peut utiliser de l'acide chromique (anodisation chromique), de l'acide sulfurique (anodisation sulfurique), de l'acide orthophosphorique, de l'acide oxalique ou un mélange de ceux-ci. Les acides peuvent également être utilisés en mélange avec un alcool tel que le mélange méthanol / NaNO₃. De préférence toutefois, la solution électrolytique comprend de l'acide sulfurique (anodisation sulfurique) ou de l'acide orthophosphorique, de manière préférée de l'acide orthophosphorique.
Dans le cas des matériaux métalliques, à l'échelle du laboratoire, on préférera mettre en oeuvre l'étape a) d'oxydation par traitement chimique, tandis qu'à l'échelle industrielle, on préférera mettre en oeuvre l'étape a) d'oxydation par traitement anodique.

### Monomères

Le monomère utilisé dans l'étape b), impliqué dans la polymérisation radicalaire, est porteur d'une fonction permettant une polymérisation radicalaire. De préférence, ledit monomère est une oléfine.
La structure des monomères utilisés dans la présente invention permet la formation d'un polymère, de préférence bioactif, à la surface d'un matériau métallique. En particulier, afin d'améliorer les propriétés d'ostéointégration et anti-bactériennes des matériaux cités ci-dessus, les monomères utilisés dans le cadre du présent procédé comprennent avantageusement un groupement sulfonate et/ou carboxylate et/ou phosphonate. En effet, les polymères porteurs de groupements ioniques sulfonates et/ou carboxylates et/ou phosphonates favorisent l'adhérence, la colonisation et la différenciation des ostéoblastes. De plus, les polymères porteurs de groupe sulfonates et/ou phosphonates permettent d'inhiber l'adhérence de souches bactériennes, notamment de *Staphylococcus aureus et de Staphylococcus epidermidis,* souches très majoritairement impliquées dans les infections sur matériels prothétiques métalliques.
Ainsi, avantageusement, l'oléfine est choisie parmi les oléfines de formule (I), (II) ou (III):

CH₂=CR₁-(CH₂)ₙ-R'-C(O)OR (I),

CH₂=CR₁-(CH₂)ₘ-R'-S(O)₂OX (II),

CH₂=CR₁-(CH₂)ₚ-R'-P(O)O₂Y (III),

dans lesquelles,
R₁ représente un atome d'hydrogène ou un alkyle en C₁-C₁₀, de préférence un atome d'hydrogène,
n est compris entre 0 et 6, de préférence entre 0 et 1,
R représente un atome d'hydrogène, un alkyle en C₁-C₁₀ optionnellement substitué par un groupe OH, COOH ou PO₃H, un groupe Ar optionnellement substitué par un groupe OH, COOH ou PO₃H, Ar représentant un groupe phényle ou hétéroaryle, de préférence un groupe phényle, ou R représente un atome d'hydrogène, ou un cation choisi parmi les alcalins ou les alcalinoterreux, par exemple parmi Na⁺, Ca²⁺, Zn²⁺ ou Mg²⁺, de préférence Na⁺ ou Ca²⁺, de préférence R représente un atome d'hydrogène, ou un cation choisi parmi les alcalins ou les alcalinoterreux, par exemple parmi Na⁺, Ca²⁺, Zn²⁺ ou Mg²⁺, de préférence Na⁺ou Ca²⁺,
m est compris entre 0 et 6, de préférence entre 0 et 1,
R' représente une liaison, un alkyle en C₁-C₁₀ optionnellement substitué par un groupe OH, COOH ou PO₃H, ou R' représente un groupe -C(O)-CR₂-OCR₃, Ar', Ar'-O-, ou Ar'-C(O)NH-, Ar' représentant un groupe phényle ou hétéroaryle optionnellement substitué par un groupe OH, COOH ou PO₃H, de préférence un groupe phényle optionnellement substitué par un groupe OH, COOH ou PO₃H, et
R₂ et R₃ représentent indépendamment des groupes alkyle en C₁-C₁₀,
X représente un hydrogène ou un ou plusieurs cations choisis de manière à obtenir une espèce électriquement neutre, notamment un cation choisi parmi les alcalins ou les alcalinoterreux, par exemple parmi 2 Na⁺, Ca²⁺, Zn²⁺ ou Mg²⁺, de préférence 2 Na⁺ou Ca²⁺,
p est compris entre 0 et 6, de préférence entre 0 et 1,
Y représente un hydrogène ou un ou plusieurs cations choisis de manière à obtenir une espèce électriquement neutre, un cation choisi parmi les alcalins ou les alcalinoterreux, par exemple parmi Na⁺, Ca²⁺, Zn²⁺ ou Mg²⁺, de préférence Na⁺ou Ca²⁺. Parmi les oléfines de formule (I), on peut notamment citer l'acide acrylique (AA), l'acide méthacrylique (MA), l'acide éthacrylique (EA), les sels correspondants (en particulier sels de métal alcalin, de préférence de sodium) et leurs mélanges.
Dans un mode de réalisation particulier, l'oléfine de formule (III) est une oléfine de formule (IV) :

CH₂=CR₁-(CH₂)_{q}-P(O)O₂Y (IV)

dans laquelle R₁ et Y sont tels que définis ci-dessus, et q est compris entre 0 et 6, de préférence entre 1 et 5, par exemple égal à 2.
Dans les modes de réalisation où une oléfine de formule (I) avec un groupe R représentant un alkyle en C₁-C₁₀ optionnellement substitué par un groupe OH, l'étape b) de polymérisation est de préférence suivie d'une étape d'hydrolyse de la fonction ester pour libérer l'acide correspondant, soit sous forme d'acide soit sous forme de sel, de préférence avec un cation choisi parmi les alcalins ou les alcalinoterreux, par exemple parmi Na⁺, Ca²⁺, Zn²⁺ ou Mg²⁺, de préférence Na⁺ ou Ca²⁺.

Parmi les oléfines de formule (II), sont particulièrement préférées les oléfines pour lesquelles R' représente un groupe Ar, Ar-O-, ou Ar-C(O)NH-, de manière encore préférée Ar , Ar représentant un groupe phényle ou hétéroaryle, de préférence un groupe phényle.
Ainsi, dans un mode de réalisation particulier, les oléfines de formule (II) sont choisies parmi les oléfines de formule (V) et (VI):

CH₂=CR₁-Ph-S(O)₂OX (V),

CH₂=CR₁-Ph-CO-NH-S(O)₂OX (VI),

dans lesquelles X et R₁ sont tels que définis ci-dessus, et Ph représente un noyau phényle, de préférence substitué en positions 1 et 4. Parmi les monomères de formule (II), (V) ou (VI), on peut notamment citer l'acrylamide de N- (sulfonate de sodium phényl) (NaAS), le méthacrylamide de N-(sulfonate de sodium phényl) (NaMS), le styrène sulfonate de sodium (NaSS) et leurs mélanges. On préfère le styrène sulfonate de sodium.
Parmi les monomères de formule (III) on peut notamment citer le vinylbenzylphosphonate (VBP) et le 2-[4-(dihydroxyphosphoryl)-2-oxa-butyl]acrylate d'éthyle (MA154).
Ainsi, avantageusement, le monomère, en particulier l'oléfine, est choisi parmi les sulfonates, les phosphonates et/ou les carboxylates, de préférence choisi parmi l'acide acrylique, l'acide méthacrylate, le méthacrylate de méthyle (MMA), l'acrylamide de N-(sulfonate de sodium phényl) (NaAS), le méthacrylamide de N-(sulfonate de sodium phényl) (NaMS), le styrène sulfonate de sodium (NaSS), l'ethylène glycol methacrylate, le méthacrylate phosphate, le methacryloyl-di-isopropylidene, le vinylbenzylphosphonate (VBP), 2-[4-(dihydroxyphosphoryl)-2-oxa-butyl]acrylate d'éthyle (MA154), ou leurs mélanges.

### Greffage par polymérisation radicalaire (étape b)

Dans la présente invention, l'étape d'amorçage par irradiation UV permet de générer des radicaux -O·. Ainsi, l'étape d'amorçage par irradiation UV consiste en une rupture homolytique de la liaison O-O du groupe hydroperoxyde (généré grâce à l'étape a) d'oxydation) ou de la liaison O-H du groupe hydroxyde (résultant de l'oxydation native du matériau) se trouvant à la surface du matériau métallique oxydé : -OOH → -O· ou - OH → -O·.
En particulier, dans le cas d'un matériau métallique en titane, le mécanisme de cette étape d'amorçage par irradiation UV peut être représenté comme suit :

TiO-OH → TiO·.

L'étape d'amorçage peut être effectuée préalablement ou de manière concomitante à l'étape de propagation.
Dans l'étape b), le (ou les) monomère(s) peut(vent) être utilisé(s) soit en solution, soit pur(s) (ou essentiellement pur(s)). Lorsque le (ou les) monomère(s) est (sont) utilisé(s) en solution, il(s) l'est (le sont) dans un solvant organique ou aqueux. De préférence, il s'agit d'une solution aqueuse.
Avantageusement la concentration de monomère(s) dans la solution est comprise entre 0.2 et 1 mol.L⁻¹, de préférence entre 0.3 et 1 mol.L⁻¹, par exemple 0.7 mol.L⁻¹. De préférence, le (ou les) monomère(s) est (sont) utilisé(s) en solution aqueuse, à une concentration comprise entre 0.2 et 1 mol.L⁻¹, de préférence entre 0.3 et 1 mol.L⁻¹, par exemple 0.7 mol.L⁻¹.
Avantageusement, ladite polymérisation radicalaire est réalisée en l'absence d'oxygène. En effet, la présence d'oxygène tend à inhiber la polymérisation radicalaire. Ainsi, de préférence, l'étape b) est réalisée en l'absence d'oxygène, sous atmosphère inerte, en particulier sous argon, hélium ou azote, avantageusement sous atmosphère d'argon.

Dans un mode de réalisation particulier, l'étape b) de greffage comprend les sous-étapes suivantes :
b1) mise en contact du matériau métallique oxydé avec ledit monomère en solution aqueuse ;
b2) irradiation UV de la solution comprenant ledit monomère et ledit matériau métallique oxydé avec une source UV diffusant une puissance à la surface du matériau supérieure à 72 mW.cm⁻², de préférence comprise entre 72 mW.cm⁻² et 20 W.cm⁻², de préférence entre 72 mW.cm⁻² et 226 mW.cm⁻², de préférence entre 91 mW.cm⁻² et 226 mW.cm⁻², de manière préférée entre toutes égale à environ 162 mW.cm⁻².
Ainsi, dans ce mode de réalisation, l'étape d'amorçage est effectuée de manière concomitante à la propagation (étape b2). Les étapes d'amorçage et de propagation ont donc lieu toutes deux lors de l'étape b2).
La solution aqueuse de l'étape b1) contenant ledit monomère, comprend en outre avantageusement un activateur UV de préférence choisi parmi la fluoresceine et la benzophénone.
Dans un autre mode de réalisation, l'étape b) de greffage comprend les sous-étapes suivantes :
b1') trempage du matériau métallique oxydé dans le monomère, ledit monomère étant essentiellement pur, conduisant à un matériau métallique oxydé imprégné de monomère ;
b2') irradiation UV du matériau métallique oxydé enrobé de monomère avec une source UV diffusant une puissance à la surface du matériau supérieure à 72 mW.cm⁻², , de préférence comprise entre 72 mW.cm⁻² et 20 W.cm⁻² de préférence entre 72 mW.cm⁻² et 226mW.cm⁻², de préférence entre 91 mW.cm⁻² et 226 mW.cm⁻², de manière préférée entre toutes égale à environ 162 mW.cm⁻².
Ainsi, dans ce mode de réalisation également, l'étape d'amorçage est effectuée de manière concomitante à la propagation (étape b2')). Les étapes d'amorçage et de propagation ont donc lieu toutes deux lors de l'étape b2').
L'étape b) comprend une irradiation UV du matériau métallique oxydé avec une source UV de longueur d'onde avantageusement comprise entre 10 nm et 380 nm, de préférence entre 200 nm et 380 nm. Par exemple, la source UV possède une longueur d'onde de 365 nm ou de 254 nm, de préférence 365 nm.
Un paramètre important de l'irradiation est sa puissance surfacique sur la surface du matériau métallique oxydé, qui est elle-même fonction de la puissance de la source UV, et de la distance d_{S-Mox} entre la source UV et le matériau métallique oxydé.
De préférence, la puissance de la source UV est comprise entre 50 et 400 W, de manière encore préférée entre 150 et 200 W.
Par exemple, lorsque la puissance de la source UV est de 200 W, la distance d_{S-Mox} peut être comprise entre 5 et 30 cm, de préférence entre 5 et 20 cm.
Dans un mode de réalisation particulier, la distance d_{S-Mox} est de 10 cm et la puissance surfacique de la source UV à 200 W est de 162 mW/cm⁻².
Ainsi, l'irradiation UV est de préférence effectuée avec une source UV diffusant une puissance surfacique sur la surface du matériau métallique oxydé supérieure à 72 mW.cm⁻², de préférence comprise entre 72 mW.cm⁻² et 20 W.cm⁻², de préférence entre 72 mW.cm⁻² et 226 mW.cm⁻², de préférence entre 91 mW.cm⁻² et 226mW.cm⁻², de manière préférée entre toutes égale à environ 162 mW.cm⁻².
Un autre paramètre important à prendre en considération est la durée d'irradiation UV t_{UV}. L'homme du métier choisira le temps nécessaire à l'irradiation du matériau métallique en fonction de la nature de ce dernier, du polymère à greffer et de la densité de greffage souhaitée. De préférence, la durée d'irradiation est inférieure à 180 minutes.

Avantageusement, la durée d'irradiation UV t_{UV} est inférieure à 120 minutes. La durée d'irradiation UV t_{UV} est par exemple comprise entre plus de 15 minutes et 120 minutes, de manière encore préférée entre 30 minutes et 60 minutes, par exemple 60 minutes.

Dans un mode de réalisation particulier dans lequel le matériau métallique est un matériau en titane, la durée d'irradiation UV t_{UV} est avantageusement comprise entre plus de 15 minutes et 120 minutes pour une puissance surfacique supérieure à 72 mW.cm⁻² de préférence.
Dans un mode de réalisation particulier dans lequel le matériau métallique est un matériau en alliage de titane, la durée d'irradiation UV t_{UV} est avantageusement comprise entre plus de 15 minutes et 120 minutes, avec une puissance surfacique supérieure à 72 mW.cm⁻² de préférence.
Avantageusement, il semble donc que le taux de greffage du matériau métallique oxydé lors de l'étape b) est fonction à la fois de la puissance d'irradiation UV reçue par le matériau métallique oxydé, et l'énergie surfacique reçue par ledit matériau.
Ainsi, avantageusement, la puissance surfacique due à l'irradiation UV reçue par le matériau métallique oxydé est comprise entre 72 et 226 mW/cm⁻², de manière encore préférée entre 91 et 162 mW/cm⁻², et l'énergie surfacique totale reçue de l'irradiation UV par le matériau métallique, de préférence prothétique, oxydé est comprise entre 194,4et 1749,6 J.cm⁻², de manière encore préférée entre 220 et 450 J.cm⁻².

L'étape b) de greffage conduit ainsi à un matériau métallique greffé.

### Etape de rinçages et de lavages dite de « conditionnement »

Le matériau métallique greffé subit différents rinçages et lavages dans des solutions aqueuses et/ou salines aqueuses à température ambiante ou à 37°C ou à 60°C. Les solutions aqueuses ou salines aqueuses sont l'eau distillée, des solutions aqueuses de NaCl de différentes concentrations et/ou une solution tampon phosphate salin (PBS ou « Phosphate-buffered saline »).
Une telle étape permet avantageusement de débarrasser le matériau métallique greffé de toute chaîne polymérique non greffée située en surface dudit matériau. Le matériau métallique greffé et rincé ainsi obtenu, une fois implanté, offre une meilleure sécurité pour le patient puisqu'elle empêche le relargage « in situ » de produits d'extraction issus de la synthèse.

### Matériaux métalliques greffés

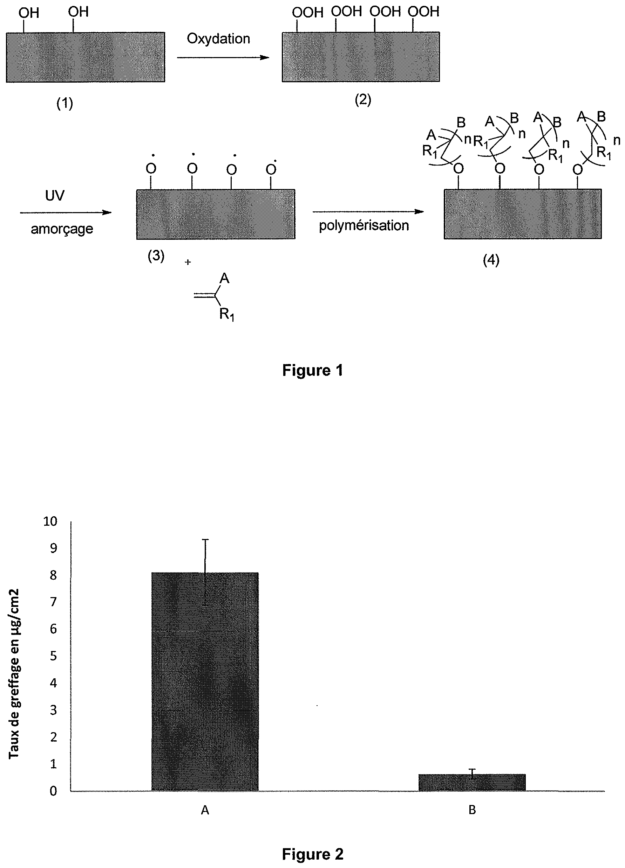

Le procédé selon l'invention peut être représenté schématiquement par la figure 1.
La figure 1 illustre bien le fait que le procédé selon l'invention est un greffage « direct » : aucune molécule intermédiaire n'est greffée à la surface du matériau métallique pour jouer le rôle de « coupleur » entre la surface du matériau et le polymère. Dans le procédé selon l'invention, le polymère est lié à la surface du matériau métallique par un simple atome d'oxygène.
Ainsi, les matériaux métalliques greffés comprennent des polymères, de préférence bioactifs, greffés à leur surface.
Les matériaux métalliques greffés obtenus par le procédé de l'invention présentent avantageusement un angle de contact avec une goutte d'eau inférieur à 50°, de préférence inférieur 45°, de façon plus préférée inférieur à 30° et pouvant être avantageusement inférieur à 20°.
Les matériaux métalliques greffés obtenus par le procédé de l'invention présentent généralement un angle de contact avec une goutte d'eau supérieur à 5°.

Les inventeurs ont notamment découvert que l'utilisation d'une irradiation UV selon l'invention permet d'obtenir une réduction importante de l'angle de contact par rapport aux matériaux métalliques non greffés. Cette découverte permet d'envisager des matériaux métalliques greffés avec des polymères présentant des angles de contact faibles et donc une bonne biocompatibilité.

Avantageusement, le procédé selon l'invention conduit à un matériau métallique greffé possédant un taux de greffage dudit polymère (de préférence bioactif) supérieur à 1,5 µg/cm⁻², de préférence supérieur à 3 µg/cm⁻².
Ainsi le procédé selon l'invention conduit à un matériau métallique greffé possédant un taux de greffage dudit polymère (de préférence bioactif) de préférence compris entre 1,5 et 10 µg.cm⁻² , de préférence entre 3 et 8 µg.cm⁻².
Le taux de greffage du polymère est dans une large mesure fonction du temps d'exposition à l'irradiation UV. Il peut dépendre également de la nature et de la quantité du monomère utilisé.
Les polymères greffés à la surface des matériaux métalliques obtenus selon le procédé de l'invention sont issus de la polymérisation radicalaire des monomères décrits plus haut, utilisés dans l'étape b).

Le poids moléculaire des polymères greffés selon le procédé de la présente l'invention peut varier dans une large mesure et est choisi ou contrôlé par l'homme du métier en fonction de leur application ou usage ultérieur. Avantageusement, le poids moléculaire moyen en poids des polymères greffés peut varier de 200 à 100 000 daltons.
De préférence, les polymères greffés à la surface des matériaux métalliques obtenus selon le procédé de l'invention sont issus de la polymérisation radicalaire d'au moins un monomère choisi parmi : l'acide acrylique, l'acide méthacrylique, le méthacrylate de méthyle (MMA), l'acrylamide de N-(sulfonate de sodium phényl) (NaAS), le méthacrylamide de N-(sulfonate de sodium phényl) (NaMS), le styrène sulfonate de sodium (NaSS), l'éthylène glycol méthacrylate phosphate, le vinylbenzylphosphonate (VBP) et le 2-[4-(dihydroxyphosphoryl)-2-oxa-butyl]acrylate d'éthyle (MA154), et leurs mélanges.
En fonction des monomères utilisés, les polymères greffés peuvent être des homopolymères ou des copolymères.
Dans un premier mode de réalisation particulier de l'invention, les polymères greffés sont des homopolymères. Dans ce mode de réalisation, un seul monomère est utilisé dans l'étape b) de polymérisation radicalaire, ledit monomère est alors de préférence une oléfine de formule (I), (II) ou (III), de préférence choisie parmi le styrène sulfonate de sodium (le polymère greffé est alors le polyNaSS) ou le méthacrylate de méthyle (le polymère greffé est alors poly (méthacrylate de méthyle), ou PMMA), le 2-[4-(dihydroxyphosphoryl)-2-oxa-butyl]acrylate d'éthyle (MA154) ou le vinylbenzylphosphonate (VBP).
Dans un autre mode de réalisation particulier de l'invention, les polymères greffés sont des copolymères. De préférence, les copolymères greffés sont obtenus par polymérisation radicalaire d'au moins deux monomères choisis parmi les oléfines de formule (I), (II) et (III) telles que définies ci-dessus. De manière encore préférée, les copolymères greffés sont obtenus par polymérisation radicalaire d'au moins deux monomères choisis parmi les oléfines de formule (I), (V) et (III) telles que définies ci-dessus.
Les quantités des oléfines de formules (I), (II) et/ou (III) peuvent varier dans une large mesure et sont adaptées en fonction notamment des propriétés désirées pour les copolymères.
Les copolymères greffés selon le procédé de la présente invention peuvent être obtenus par polymérisation radicalaire de monomères comprenant, en plus des monomères de formules (I), (II) et (III), d'autres monomères de type oléfine. Les oléfines additionnelles peuvent être de toute nature, avantageusement des oléfines donnant un caractère hydrosoluble ou non aux polymères greffés. De préférence, les monomères additionnels sont de nature hydrosoluble, tels que les oléfines comprenant un groupe de type sucre, notamment des oléfines comprenant un groupe ose tel que le glucose, le glucofuranose, le saccharose, le fructose, le mannose.
Dans les copolymères comprenant un monomère additionnel, la quantité des monomères de formule (I), (II) et (III) est avantageusement supérieure ou égale à 25 %, de préférence supérieure ou égale à 50%, en moles par rapport au nombre total de moles des unités monomères présentes dans les polymères.
Selon un aspect préféré de l'invention, les copolymères greffés sont obtenus par polymérisation radicalaire de deux ou trois monomères choisis parmi les oléfines de formule (I), (II) et (III) telles que définies ci-dessus.
De manière préférée, le polymère greffé est le PolyNaSS.
Le matériau est de préférence abondamment lavé et stérilisé préalablement à l'implantation.

### Modes de réalisation particuliers

Dans un mode de réalisation préféré, le procédé selon l'invention est un procédé de greffage de polymères PolyNaSS sur un matériau métallique en titane ou alliage de titane (l'alliage étant avantageusement le TiAl₆V₄), comprenant les étapes suivantes :
a) oxydation de la surface du matériau en titane ou en alliage de titane, conduisant à un matériau oxydé, et
b) greffage d'un polymère PolyNaSS à la surface dudit matériau oxydé par polymérisation radicalaire du monomère styrène sulfonate de sodium (NaSS), ladite polymérisation radicalaire comprenant une étape d'amorçage, une étape de propagation,
ladite étape d'amorçage étant effectuée par irradiation UV avec une source UV diffusant une puissance à la surface du matériau supérieure à 72 mW.cm⁻², de préférence comprise entre 72 mW.cm⁻² et 20 W.cm⁻², de préférence entre 72 mW.cm⁻² et 226mW.cm⁻², de préférence entre 91 mW.cm⁻² et 226 mW.cm⁻², de manière préférée entre toutes égale à environ 162 mW.cm⁻²,
de préférence, ladite irradiation UV étant réalisée pendant une durée supérieure à 15 minutes et inférieure à 180 minutes, de préférence inférieure ou égale à 120 minutes, de manière plus préférée inférieure ou égale à 90 minutes,
ledit procédé conduisant à un matériau greffé.

Dans ce mode de réalisation particulier, l'étape b) de greffage peut comprendre les sous-étapes suivantes :
b1) mise en contact du matériau en titane ou en alliage de titane oxydé avec le styrène sulfonate de sodium (NaSS) en solution aqueuse ;
b2) irradiation UV de la solution comprenant le styrène sulfonate de sodium (NaSS) et ledit matériau en titane ou en alliage de titane oxydé avec une source UV diffusant une puissance à la surface du matériau comprise entre 72 mW.cm⁻² et 20 W.cm⁻², de préférence entre 72 mW.cm⁻² et 226 mW.cm⁻², de préférence entre 91 mW.cm⁻² et 226 mW.cm⁻², de manière préférée entre toutes égale à environ 162 mW.cm⁻².
Alternativement, l'étape b) de greffage peut comprendre les sous-étapes suivantes :
b1') trempage du matériau en titane ou en alliage de titane oxydé dans le styrène sulfonate de sodium (NaSS), ledit styrène sulfonate de sodium (NaSS) étant essentiellement pur, conduisant à un matériau métallique oxydé enrobé de styrène sulfonate de sodium (NaSS) ;
b2') irradiation UV du matériau métallique oxydé de en solution aqueuse avec une source UV diffusant une puissance à la surface du matériau supérieur à 72 mW.cm⁻², de préférence comprise entre 72mW.cm⁻² et 226 mW.cm⁻², de préférence entre 91 mW.cm⁻² et 226 mW.cm⁻², de manière préférée entre toutes égale à environ 162 mW.cm⁻².
De préférence, dans ce mode de réalisation, le procédé comprend une étape de décapage.
Il est entendu que l'homme du métier pourra combiner tous les modes de réalisation particuliers et préférés des étapes a) et b), avec le procédé de ce mode de réalisation particulier.
Ainsi, le matériau greffé obtenu par le procédé selon l'invention est de préférence un matériau (de préférence prothétique) en titane ou alliage de titane à la surface duquel sont greffés directement des polymères, du polyNaSS de préférence, avantageusement avec un taux de greffage supérieur à 1,5 µg.cm⁻² , de préférence compris entre 1,5 et 10 µg.cm⁻² , de manière encore préférée avec un taux de greffage compris entre 3 et 8 µg.cm⁻².
Le matériau est de préférence lavé et stérilisé préalablement à l'implantation.

### Matériau susceptible d'être obtenu par le procédé

La présente invention concerne également les matériaux métalliques susceptibles d'être obtenus par le procédé selon l'invention.
Les matériaux métalliques susceptibles d'être obtenus par le procédé selon l'invention sont donc greffés en surface par des polymères, de préférence bioactifs.
Les matériaux métalliques greffés susceptibles d'être obtenus par le procédé de l'invention présentent avantageusement un angle de contact inférieur à 50°, de préférence inférieur à 45°, de façon plus préférée inférieur à 40°, encore plus avantageusement inférieur à 30° pouvant même être inférieur à 20° Les matériaux métalliques greffés obtenus par le procédé de l'invention présentent généralement un angle de contact avec une goutte d'eau supérieur à 5°.

Les matériaux métalliques greffés susceptibles d'être obtenus par le procédé selon l'invention possèdent un taux de greffage supérieur à 1,5 µg/cm⁻², de préférence supérieur à 3 µg/cm⁻², de préférence compris entre 1,5 et10 µg.cm⁻² , de façon plus préférée entre 3 et 10 µg.cm⁻².
Les polymères greffés à la surface des matériaux métalliques susceptibles d'être obtenus selon le procédé de l'invention sont issus de la polymérisation radicalaire des monomères décrits plus haut.
Le poids moléculaire des polymères greffés peut varier dans une large mesure et est choisi ou contrôlé par l'homme du métier en fonction de leur application ou usage ultérieur. Avantageusement, le poids moléculaire moyen en poids des polymères greffés peut varier de 200 à 100 000 daltons.
De préférence, les polymères greffés à la surface des matériaux métalliques susceptibles d'être obtenus selon le procédé de l'invention sont issus de la polymérisation radicalaire d'au moins un monomère choisi parmi : l'acide acrylique, l'acide méthacrylique, le méthacrylate de méthyle (MMA), l'acrylamide de N-(sulfonate de sodium phényl) (NaAS), le méthacrylamide de N-(sulfonate de sodium phényl) (NaMS), le styrène sulfonate de sodium (NaSS), l'éthylène glycol méthacrylate phosphate, le vinylbenzylphosphonate (VBP) et le 2-[4-(dihydroxyphosphoryl)-2-oxa-butyl]acrylate d'éthyle (MA154).
En fonction des monomères utilisés, les polymères greffés peuvent être des homopolymères ou des copolymères.
Dans un premier mode de réalisation particulier de l'invention, les polymères greffés sont des homopolymères. Dans ce mode de réalisation, un seul monomère est utilisé dans la polymérisation radicalaire, ledit monomère est alors de préférence une oléfine de formule (I), (II), (III) ou (IV) telle que définie ci-dessus, de préférence choisie parmi le styrène sulfonate de sodium (le polymère greffé est alors le polyNaSS) ou le méthacrylate de méthyle (le polymère greffé est alors poly (méthacrylate de méthyle), ou PMMA), le Vinylbenzylphosphonate (VBP) ou le 2-[4-(dihydroxyphosphoryl)-2-oxa-butyl]acrylate d'éthyle (MA154).
Dans un autre mode de réalisation particulier de l'invention, les polymères greffés sont des copolymères. De préférence, les copolymères greffés sont obtenus par polymérisation radicalaire d'au moins deux monomères choisis parmi les oléfines de formule (I), (II), (III) et (IV) telles que définies ci-dessus. De manière encore préférée, les copolymères greffés sont obtenus par polymérisation radicalaire d'au moins deux monomères choisis parmi les oléfines de formule (I), (II) et (III) telles que définies ci-dessus.
Les quantités des oléfines de formules (I), (II), (III) et/ou (IV) peuvent varier dans une large mesure et sont adaptées en fonction notamment des propriétés désirées pour les copolymères.
Les copolymères greffés selon le procédé de la présente invention peuvent être obtenus par polymérisation radicalaire de monomères comprenant, en plus des monomères de formules (I), (II), (III) et (IV), d'autres monomères de type oléfine. Les oléfines additionnelles peuvent être de toute nature, avantageusement des oléfines donnant un caractère hydrosoluble aux polymères greffés. De préférence, les monomères additionnels sont de nature hydrosoluble, tels que les oléfines comprenant un groupe de type sucre, notamment des oléfines comprenant un groupe ose tel que le glucose, le glucofuranose, le saccharose, le fructose, le mannose.
Dans les copolymères comprenant un monomère additionnel, la quantité des monomères de formule (I), (II), (III) et (IV) est avantageusement supérieure ou égale à 25 %, de préférence supérieure ou égale à 50%, en moles par rapport au nombre total de moles des unités monomères présentes dans les polymères.
Selon un aspect préféré de l'invention, les copolymères greffés sont obtenus par polymérisation radicalaire de deux ou trois monomères choisis parmi les oléfines de formule (I), (II), (III) et (IV) telles que définies ci-dessus.
De manière préférée, les polymères greffés sont le PolyNaSS, le vinylbenzylphosphonate (VBP) et le 2-[4-(dihydroxyphosphoryl)-2-oxa-butyl]acrylate d'éthyle (MA154).
Le matériau est de préférence lavé et stérilisé préalablement à l'implantation.

### Utilisations des matériaux selon l'invention

Un implant prothétique fabriqué à partir des matériaux métalliques susceptibles d'être obtenus par le procédé de l'invention.
Les implants prothétiques sont avantageusement des implants articulaires, en particulier utiles en tant que prothèse de hanche, ou des implants dentaires
La présente demande divulgue également des implants pour leur utilisation dans le remplacement d'une articulation ou d'une dent, notamment par chirurgie.

### DESCRIPTION DES FIGURES

La figure 1 représente schématiquement un mode de réalisation du procédé selon l'invention, faisant intervenir les différentes espèces intermédiaires mises en jeu à chaque étape du procédé. Le matériau (1) correspond au matériau métallique prothétique non traité avec une couche d'oxydation en surface à l'état natif. Le matériau (2) a subi l'étape a) d'oxydation, et présente en surface des fonctions hydroperoxydes, avec une densité équivalente ou plus importante que les fonctions OH du matériau à l'état natif. Le matériau (3) a subi une étape d'amorçage par irradiation UV : une rupture homolytique de la liaison O-O ou OH a eu lieu, donnant naissance à des radicaux O en surface. Ce matériau amorcé (3) est ensuite mis en présence d'une oléfine de formule CH₂=CR₁A (dans laquelle par exemple R₁ représente H, et A représente Ph-SO₃Na), qui subit l'étape de polymérisation, pour conduire au matériau greffé (4).
La figure 2 est un diagramme à barres démontrant l'effet de l'étape a) d'oxydation sur le taux de greffage d'un matériau prothétique en titane (exemple 3). L'axe des ordonnées représente le taux de greffage en µg.cm⁻². La barre de gauche (A) représente le taux de greffage moyen obtenu pour 6 échantillons de matériau en titane ayant subi les étapes a) et b) du procédé selon l'invention, et la barre de droite (B) représente le taux de greffage moyen obtenu pour 2 échantillons de matériau en titane ayant subi uniquement l'étape b) du procédé selon l'invention (pas d'oxydation contrôlée, seulement l'oxydation naturelle).

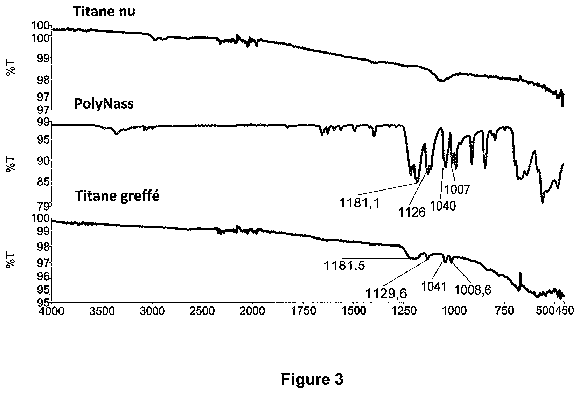

La figure 3 représente trois spectres infrarouges : spectre du du titane brut (matériau prothétique non traité i.e. à l'état natif, spectre du haut), spectre du du polyNaSS non greffé (spectre du milieu), et spectre du d'un matériau en titane greffé avec du polyNaSS selon le procédé de l'exemple 1 (spectre du bas). L'axe des ordonnées représente la transmittance (en %). L'axe des abscisses représente le nombre d'onde (en cm⁻¹).

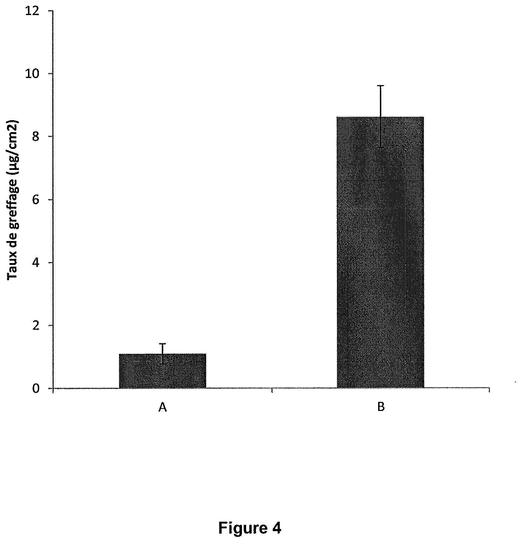

La figure 4 est un diagramme à barres démontrant l'impact du temps d'attente entre l'étape de décapage et l'étape d'oxydation. L'axe des ordonnées représente le taux de greffage en µg.cm⁻². La barre de gauche (A) représente le taux de greffage moyen obtenu pour un matériau en titane ayant subi l'étape d'oxydation 16 heures après l'étape de décapage, et la barre de droite (B) représente le taux de greffage moyen obtenu pour un matériau en titane ayant subi l'étape d'oxydation 2 heures après l'étape de décapage.

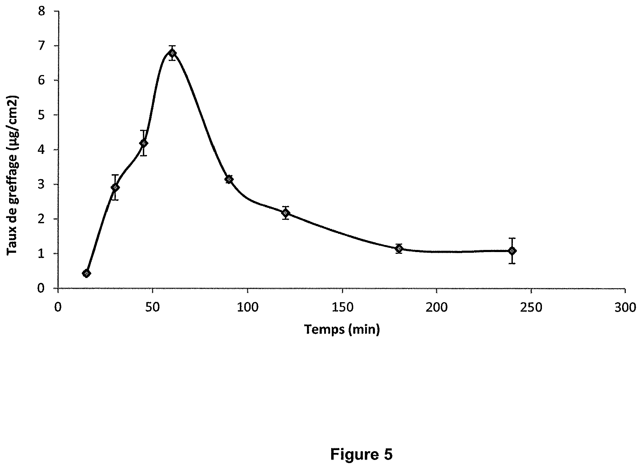

La figure 5 représente le taux de greffage d'un matériau métallique en titane de l'exemple 4 en fonction du temps d'irradiation UV du matériau oxydé dans une solution à 0.32 M en monomère. L'axe des ordonnées représente le taux de greffage en µg.cm⁻², et l'axe des abscisses représente le temps d'irradiation UV en minutes. Les valeurs représentées sont des moyennes sur trois expériences.

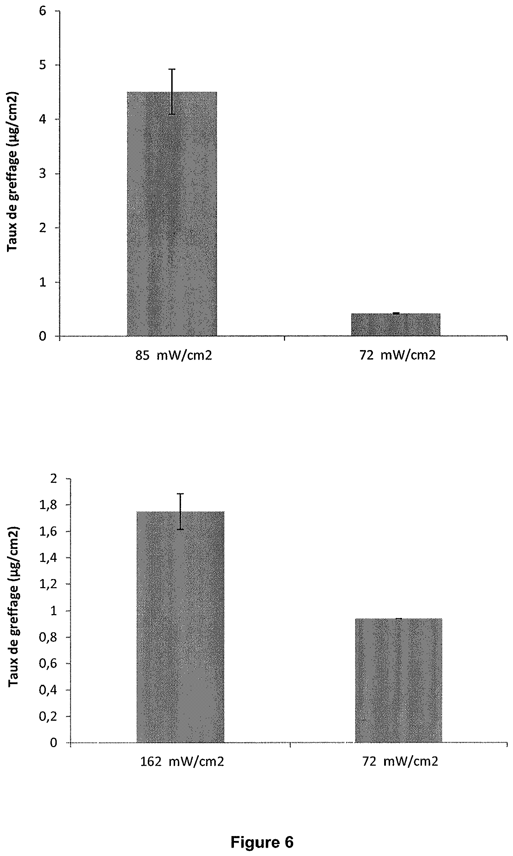

La figure 6 représente le taux de greffage d'un matériau métallique en titane en fonction de la puissance surfacique de l'irradiation UV, pour un temps d'exposition de 45min sur un matériau en alliage de titane (Figure 6A) et sur un matériau en titane (figure 6B), dans une solution à 0.7 M en monomère. Les valeurs représentées sont des moyennes sur trois expériences.

### EXEMPLES

Les avantages de la présente invention apparaîtront à la lumière des exemples suivants, qui concernent des modes de réalisation particuliers de l'invention, mais ne sauraient être considérés comme limitatifs.

### Exemple 1 : Mise en œuvre du procédé selon l'invention

### 1.1. Mise en œuvre du procédé

Le matériau métallique est dans cet exemple un matériau en titane ou en alliage de titane.

### 1.1.1 Polissage

Les surfaces du matériau métallique en titane peuvent être tout d'abord polies.
Le polissage mécanique des disques de titane est réalisé à l'aide d'un bras automatique, monté sur une polisseuse rotative, avec du papier abrasif de granulométries décroissantes (Struers). Un premier polissage avec un papier de grade 500 (ci-après P500) enlève environ 1/10^{ème} de millimètres d'épaisseur. Le polissage est ensuite affiné par l'utilisation d'un papier de granulométrie plus faible, 1200 (ci-après P1200).
Le protocole utilisé est le suivant : les disques sont polis 1 à 2 minutes avec du papier P500 avec une rotation de 200 tours/minute (t/min) puis 1 à 2 minutes avec P1200.
Après le polissage, les échantillons sont lavés par trempage dans une solution d'acétone (1 nuit) puis 2x15min dans l'acétone au bain à ultrasons, et enfin 3x15min au bain à ultrasons dans l'eau distillée. Ils sont ensuite séchés à 60°C et utilisés immédiatement ou conservés sous argon.

### 1.1.2 Décapage

Le matériau métallique poli subit ensuite une étape de décapage dans un mélange H₂O/HF/HNO₃ (88/2/10). La solution utilisée pour ce lavage est un mélange d'eau, d'une solution aqueuse d'acide fluorhydrique à 24 M et d'une solution aqueuse d'acide nitrique à 10 M de proportions respectives (88/2/10) (v/v/v), agité pendant 1 minute. Les échantillons sont ensuite séchés à l'étuve à 60°C.

### 1.1.3 Oxydation

Le matériau métallique décapé est ensuite soumis à une étape d'oxydation par traitement chimique. Le matériau métallique est ainsi immergé dans un mélange d'acide sulfurique concentré H₂SO₄ et de peroxyde d'hydrogène H₂O₂.
Les échantillons sont immergés dans un volume v de solution d'acide sulfurique concentré H₂SO₄ (dilué à 50% avec de l'eau pour l'alliage) sous agitation pendant 1 minute. Un volume équivalent v de peroxyde d'hydrogène H₂O₂ (30% en volume d'eau) est additionné et les échantillons sont laissés dans ce mélange sous agitation pendant 3 minutes. Les surfaces oxydées sont ensuite rincées dans 3 bains d'eau pendant 3 minutes.
Alternativement, le matériau métallique décapé peut être soumis à une étape d'oxydation par traitement anodique.

### 1.1.4 Greffage

Le matériau métallique oxydé est ensuite immergé dans une solution aqueuse dégazée de monomère styrène sulfonate de sodium (NaSS) à 0.7 mol/L ou 0,32 mol/L. La solution dans laquelle est immergée le matériau oxydé est ensuite exposée pendant un temps variant entre 15 min et 240 min en fonction des échantillons à des irradiations UV provenant d'une source UV de longueur d'onde 365 nm et de 200 W de puissance. En fonction de la distance entre la lampe et le matériau métallique (de 5 cm à 30 cm), la puissance surfacique varie entre 72 mW.cm⁻² et 226 mW.cm⁻².

### 1.2. Caractérisation :

La présence des polymères greffés à la surface a été mesurée par différentes méthodes.

### 1.2.1. Méthode colorimétrique au bleu de toluidine (BT)

Les échantillons métalliques greffés sont mis au contact d'une solution de BT à 5.10⁻⁴ M (ajustée à pH 10 grâce à de la soude) à la température de 30°C pendant 6heures. Cette étape correspond à la complexation du BT aux unités monomères du polymère greffé. Les échantillons sont ensuite rincés abondamment avec une solution d'hydroxyde de sodium 1.10⁻³M pour éliminer le BT non complexé. On arrête les rinçages lorsque la solution est incolore. Le BT complexé est ensuite décomplexé au moyen d'une solution d'acide acétique 50% qui est laissée au contact des échantillons de titane pendant 24 heures. La solution obtenue est dosée par spectrophotométrie à l'aide d'un spectrophotomètre Perkin Elmer Lambda 25 (Biomacromolecules 2006, 7, 755-760).

### 1.2.2. Infrarouge à Transformée de Fourier en réflexion totale atténuée (ATR-FTIR).

Les échantillons sont directement analysés (sans préparation) par ATR-FTIR sur un appareil Perkin Elmer, spectrum Two.

### 1.2.3. Mesure d'angle de contact

Les mesures des angles de contact se font à partir d'une goutte d'eau déposée à la surface des échantillons oxydé ou greffé via l'appareil KRUSS : DSA100 qui permet de connaître les modifications du caractère hydrophile ou hydrophobe de la surface.

### 1.3. Résultats

### 1.3.1 Validation du greffage

Pour les échantillons obtenus par mise en oeuvre du procédé décrit au point 1.1., les bandes caractéristiques du groupement sulfonate à 1180 cm⁻¹ et 1128 cm⁻¹, ainsi que le doublet de vibrations à 1010 1040 cm⁻¹, la vibration symétrique à 1040 cm⁻¹ sont observés dans le spectre infrarouge en réflexion totale atténuée (ATR-FTIR, voir figure 3).

### 1.3.2 Importance de l'étape d'oxydation

La mesure de la quantité de polymères polyNaSS greffés à la surface du titane a été réalisée en complexant les groupements sulfonate des polymères avec du bleu de toluidine, à la fois sur des échantillons ayant subi le procédé décrit au point 1.1 (e.g. polissage, décapage, oxydation), mais également sur des échantillons de matériau métallique n'ayant pas subi une étape d'oxydation (e.g. seulement polissage et décapage).

Ainsi, pour les échantillons ayant subi la totalité du procédé décrit au point 1.1., un taux de greffage de l'ordre de 8 µg/cm² est observé (voir la figure 2) alors que les échantillons n'ayant pas subit l'étape d'oxydation contrôlée présente un taux de greffage de 0,62 µg/cm⁻².
L'oxydation du matériau métallique par oxydation chimique aussi bien que l'oxydation anodique donnent des résultats satisfaisants.
Il est à noter qu'un échantillon de matériau en titane oxydé immergé dans une solution de polyNaSS (e.g. Acros, Mn= 70000g/mol, n°lot : A012503701, CAS : 24704-18-1) à une concentration de 0.7 mol/L puis abondamment lavé par rinçage à l'eau, mais non soumis à une étape de greffage proprement dite, donne des valeurs de 0,3 µg/cm² par mesure par complexation au bleu de toluidine. Cette expérience permet d'écarter l'hypothèse d'une simple adsorption du polymère à la surface du matériau métallique (e.g. en titane) oxydé.
En conclusion, l'étape d'oxydation est essentielle à l'obtention d'un matériau métallique greffé selon l'invention. Elle permet d'augmenter d'un facteur 25 (7,7 vs 0,3 µg/cm) le taux de greffage par rapport à un greffage sur une surface métallique présentant une oxydation naturelle.

### 1.3.3. Propriétés mesurées

Sur un matériau en titane « pur » (i.e. titane de départ non greffé, i.e. n'ayant pas subi le procédé de l'invention), l'angle de contact est de 59 ±5°.
Les échantillons de matériau greffé obtenus selon le procédé décrit au point 1.1. présentent une valeur d'angle de contact égale à 15 ± 2°, soit une baisse de l'angle de contact de 44° entre la surface non traitée et la surface greffée.
En conclusion, les matériaux métalliques greffés obtenus selon le procédé de l'invention possèdent une surface beaucoup plus hydrophile que les matériaux métalliques non greffés et cela est rendu possible notamment par l'étape préalable d'oxydation.

### Exemple 2 : Importance du temps entre le décapage et l'oxydation

### 2.1. Protocole

Le matériau métallique est dans cet exemple un matériau en titane. 15 échantillons dudit matériau métallique sont utilisés.

Les surfaces des 15 échantillons de matériau métallique en titane sont tout d'abord polies puis subissent ensuite une étape de décapage dans un mélange H₂O/HF/HNO₃ (88/2/10).
Les 15 échantillons décapés sont ensuite soumis à une étape d'oxydation.
Après une période d'attente variant de 2 heures à 16 heures, les 15 échantillons oxydés sont ensuite soumis à l'étape de greffage dans une solution aqueuse de monomère styrène sulfonate de sodium (NaSS).
Les autres conditions utilisées sont identiques à celles décrites dans l'exemple 1.

### 2.2. Résultats

La figure 4 montre que le temps entre la fin du décapage et le début de l'oxydation à une importance sur le taux de greffage selon la méthode de l'invention.
Si 16 heures ou plus sépare la fin de l'étape de décapage du début de l'étape d'oxydation alors le taux de greffage n'est pas optimal (e.g. 1,09 µg/cm⁻²).
Ainsi il est préférable réaliser l'étape d'oxydation assez rapidement après l'étape de décapage.

### Exemple 3 : Influence du temps d'irradiation sur le taux de greffage

### 3.1. Protocole

Le matériau métallique est dans cet exemple un matériau en titane.
15 échantillons dudit matériau métallique sont utilisés.
Les surfaces des 15 échantillons de matériau métallique en titane sont tout d'abord polies.
Les 15 échantillons subissent ensuite une étape de décapage dans un mélange H₂O/HF/HNO₃ (88/2/10).
Les 15 échantillons décapés sont ensuite soumis à une étape d'oxydation par traitement chimique.
Les 15 échantillons oxydés sont ensuite soumis à l'étape de greffage dans une solution aqueuse de monomère styrène sulfonate de sodium (NaSS).
Les autres conditions utilisées sont identiques à celles décrites dans l'exemple 1.

### 3.2. Résultats

Le taux de greffage du polyNaSS à la surface des 15 échantillons de titane a été étudié en complexant les groupements sulfonate des polymères avec du bleu de toluidine.

Les résultats présentés dans le tableau 1 et la figure 5 montrent que les résultats optimaux, i.e. un taux de greffage supérieur à 1.5 µg/cm⁻², sont obtenus dans ce cas pour une irradiation UV comprise entre 30 et 120 minutes.

**Tableau 1**

| **Temps (min)** | **Moy (µg.cm⁻²)** |
|---|---|
| 15 | 0,43 |
| 30 | 2,91 |
| 45 | 4,19 |
| 60 | 6,79 |
| 90 | 3,15 |
| 120 | 2,18 |
| 180 | 1,15 |
| 240 | 1,09 |

### Exemple 4 : Influence de la puissance surfacique d'irradiation UV sur le taux de greffage

### 4.1. Protocole

Le matériau métallique est dans cet exemple un matériau en titane ou en alliage de titane.
15 échantillons dudit matériau métallique sont utilisés.
Les surfaces des 15 échantillons de matériau métallique en titane ou en alliage de titane subissent une étape de décapage.
Les 15 échantillons décapés sont ensuite soumis à une étape d'oxydation.
Les 15 échantillons oxydés sont ensuite immergés dans une solution aqueuse de monomère styrène sulfonate de sodium (NaSS).
La solution dans laquelle est immergée le matériau oxydé est ensuite exposée pendant 45min à des distances variant entre 5 centimètres et 30 centimètres des irradiations UV provenant d'une source UV de 200W de longueur d'onde 365 nm, de puissance surfacique variant ainsi entre 72 mW.cm⁻² et 226 mW.cm⁻².
Les autres conditions utilisées sont identiques à celles décrites dans l'exemple 1.

### 4.1. Résultats

La figure 6 montre qu'une puissance surfacique supérieure à 72 mW.cm⁻² est nécessaire pour induire un greffage satisfaisant sur le matériau métallique.

## Revendications

1. Procédé de greffage direct de polymères bioactifs sur un matériau métallique prothétique en titane ou en alliage de titane, comprenant les étapes suivantes :
a) l'oxydation de la surface du matériau métallique, conduisant à une surface oxydée de matériau métallique ;
b) le greffage d'un polymère à partir de la surface oxydée dudit matériau métallique, par polymérisation radicalaire d'un monomère mis en présence de la surface oxydée du matériau métallique, ladite polymérisation radicalaire comprenant une étape d'amorçage et une étape de propagation,
ladite étape d'amorçage étant effectuée par irradiation UV avec une source UV diffusant une puissance à la surface du matériau supérieure à 72 mW.cm⁻²,
ladite irradiation UV étant réalisée pendant une durée supérieure à 30 minutes et inférieure à 180 minutes,
ledit procédé conduisant à un matériau métallique prothétique en titane ou en alliage de titane greffé de polymères bioactifs.

2. Procédé selon la revendication 1, **caractérisé en ce que** ladite étape d'amorçage est effectuée par irradiation UV avec une source UV diffusant une puissance comprise entre 72 mW.cm⁻² et 20 W.cm⁻².

3. Procédé selon la revendication 1 ou 2, **caractérisé en ce que** ladite étape d'amorçage est effectuée par irradiation UV avec une source UV pendant une durée inférieure ou égale à 90 minutes.

4. Procédé selon l'une des revendications 2 à 3, **caractérisé en ce que** ladite étape d'amorçage est effectuée par irradiation UV avec une source UV diffusant une puissance comprise entre 72 mW.cm⁻² et 226 mW.cm⁻².

5. Procédé selon l'une des revendications 1 à 4, **caractérisé en ce que** ladite étape d'amorçage est effectuée sans chauffage additionnel à l'irradiation UV.

6. Procédé selon l'une des revendications 1 à 5, **caractérisé en ce que** la concentration de monomère(s) dans la solution est comprise entre 0.2 et 1 mol.L⁻¹.

7. Procédé selon l'une des revendications 1 à 6, **caractérisé en ce que** le matériau métallique est un alliage de titane à base de nickel, vanadium, aluminium, niobium, et/ou molybdène, notamment un alliage de titane, d'aluminium et de vanadium, typiquement l'alliage Ti Al₆V₄.

8. Procédé selon l'une des revendications 1 à 7, **caractérisé en ce que** ledit monomère est une oléfine.

9. Procédé selon l'une quelconque des revendications 1 à 8, **caractérisé en ce que** le monomère est le styrène sulfonate de sodium (NaSS).

10. Procédé selon l'une quelconque des revendications 1 à 8, **caractérisé en ce que** le monomère est choisi parmi les sulfonates, les phosphonates et/ou les carboxylates, de préférence choisi parmi l'acide acrylique, l'acide méthacrylate, le méthacrylate de méthyle (MMA), l'acrylamide de N-(sulfonate de sodium phényl) (NaAS), le méthacrylamide de N-(sulfonate de sodium phényl) (NaMS), l'ethylène glycol methacrylate, le méthacrylate phosphate, le methacryloyl-di-isopropylidene, le vinylbenzylphosphonate (VBP), 2-[4-(dihydroxyphosphoryl)-2-oxa-butyl]acrylate d'éthyle (MA154), ou leurs mélanges.

11. Procédé selon l'une quelconque des revendications 1 à 10, **caractérisé en ce que** ladite polymérisation radicalaire est réalisée en l'absence d'oxygène.

12. Procédé selon l'une quelconque des revendications 1 à 11, **caractérisé en ce que** l'étape d'amorçage est effectuée préalablement ou de manière concomitante à l'étape de propagation.

13. Procédé selon l'une quelconque des revendications 1 à 12, **caractérisé en ce qu'**il comprend une étape de décapage réalisée préalablement à l'étape d'oxydation, le temps entre la fin de l'étape de décapage et le début de l'étape d'oxydation étant inférieur à 16 heures, de préférence inférieur à 12 heures.

14. Procédé selon l'une quelconque des revendications 1 à 13, **caractérisé en ce que** l'étape a) d'oxydation est réalisée par traitement du matériau avec une solution aqueuse comprenant un oxydant et un acide, de préférence un mélange H2SO4 et H2O2.

15. Procédé selon l'une quelconque des revendications 1 à 14, **caractérisé en ce que** l'étape a) d'oxydation est réalisée par traitement anodique.

16. Procédé selon l'une quelconque des revendications 1 à 15, **caractérisé en ce que** le matériau métallique greffé possède un taux de greffage dudit polymère supérieur à 1,5 µg.cm-2, de préférence supérieur à 3 µg.cm-2.

## Patentansprüche

1. Verfahren zur direkten Pfropfung von bioaktiven Polymeren auf ein prothetisches metallisches Material aus Titan oder einer Titanlegierung, umfassend die folgenden Schritte:
a) Oxidation der Oberfläche des metallischen Materials, was zu einer oxidierten Oberfläche des metallischen Materials führt;
b) Pfropfung eines Polymers von der oxidierten Oberfläche des metallischen Materials durch Radikalpolymerisation eines Monomers in Gegenwart der oxidierten Oberfläche des metallischen Materials, wobei die Radikalpolymerisation einen Initiationsschritt und einen Ausbreitungsschritt umfasst,
wobei der Initiationsschritt durch UV-Bestrahlung mit einer UV-Quelle durchgeführt wird, die an der Oberfläche des Materials eine Leistung von mehr als 72 mW·cm⁻² verteilt,
wobei die UV-Bestrahlung für eine Dauer von mehr als 30 Minuten und weniger als 180 Minuten durchgeführt wird,
wobei dieses Verfahren zu einem prothetischen metallischen Material aus Titan oder Titanlegierung führt, das mit bioaktiven Polymeren gepfropft ist.

2. Verfahren nach Anspruch 1, **dadurch gekennzeichnet, dass** der Initiationsschritt durch UV-Bestrahlung mit einer UV-Quelle durchgeführt wird, die eine Leistung zwischen 72 mW·cm⁻² und 20 mW·cm⁻² verteilt.

3. Verfahren nach Anspruch 1 oder 2, **dadurch gekennzeichnet, dass** der Initiationsschritt durch UV-Bestrahlung mit einer UV-Quelle für eine Dauer von nicht mehr als 90 Minuten durchgeführt wird.

4. Verfahren nach einem der Ansprüche 2 bis 3, **dadurch gekennzeichnet, dass** der Initiationsschritt durch UV-Bestrahlung mit einer UV-Quelle durchgeführt wird, die eine Leistung zwischen 72 mW·cm⁻² und 226 mW·cm⁻² verteilt.

5. Das Verfahren nach einem der Ansprüche 1 bis 4, **dadurch gekennzeichnet, dass** der Initiationsschritt ohne zusätzliche Erwärmung zur UV-Strahlung durchgeführt wird.

6. Verfahren nach einem der Ansprüche 1 bis 5, **dadurch gekennzeichnet, dass** die Konzentration von Monomer (en) in der Lösung zwischen 0,2 und 1 mol·l⁻¹ liegt.

7. Verfahren nach einem der Ansprüche 1 bis 6, **dadurch gekennzeichnet, dass** das metallische Material eine Titanlegierung auf der Basis von Nickel, Vanadium, Aluminium, Niob und/oder Molybdän ist, insbesondere eine Legierung aus Titan, Aluminium und Vanadium, typischerweise die Legierung TiAl₆V₄.

8. Das Verfahren nach einem der Ansprüche 1 bis 7, **dadurch gekennzeichnet, dass** das Monomer ein Olefin ist.

9. Das Verfahren nach einem der Ansprüche 1 bis 8, **dadurch gekennzeichnet, dass** das Monomer Natriumstyrolsulfonat (NaSS) ist.

10. Verfahren nach einem der Ansprüche 1 bis 8, **dadurch gekennzeichnet, dass** das Monomer ausgewählt wird aus Sulfonaten, Phosphonaten und/oder Carboxylaten, vorzugsweise ausgewählt aus Acrylsäure, Methacrylsäure, Methylmethacrylat (MMA), N-(Natriumphenylsulfonat)acrylamid (NaAS), N-(Natrium-Phenylsulfonat)-Methacrylamid (NaMS), Ethylenglykolmethacrylat, Methacrylatphosphat, Methacryloyl-di-isopropyliden, Vinylbenzylphosphonat (VBP), Ethyl-2-[4-(Dihydroxyphosphoryl)-2-oxa-butyl]acrylat (MA154) oder Mischungen derselben.

11. Verfahren nach einem der Ansprüche 1 bis 10, **dadurch gekennzeichnet, dass** die Radikalpolymerisation in Abwesenheit von Sauerstoff durchgeführt wird.

12. Das Verfahren nach einem der Ansprüche 1 bis 11, **dadurch gekennzeichnet, dass** der Initiationsschritt vor oder gleichzeitig mit dem Ausbreitungsschritt durchgeführt wird.

13. Verfahren nach einem der Ansprüche 1 bis 12, **dadurch gekennzeichnet, dass** es einen Reinigungsschritt umfasst, der vor dem Oxidationsschritt durchgeführt wird, wobei die Zeit zwischen dem Ende des Reinigungsschritts und dem Beginn des Oxidationsschritts weniger als 16 Stunden, vorzugsweise weniger als 12 Stunden, beträgt.

14. Verfahren nach einem der Ansprüche 1 bis 13, **dadurch gekennzeichnet, dass** der Oxidationsschritt a) durch Behandlung des Materials mit einer wässrigen Lösung, die ein Oxidationsmittel und eine Säure, vorzugsweise ein Gemisch aus H₂SO₄ und H₂O₂, enthält, durchgeführt wird.

15. Verfahren nach einem der Ansprüche 1 bis 14, **dadurch gekennzeichnet, dass** der Oxidationsschritt a) durch eine anodische Behandlung durchgeführt wird.

16. Das Verfahren nach einem der Ansprüche 1 bis 15, **dadurch gekennzeichnet, dass** das gepfropfte metallische Material eine Pfropfrate des Polymers von mehr als 1,5 µg·cm⁻², vorzugsweise von mehr als 3 µg·cm⁻², aufweist.

## Claims

1. A process for direct grafting of bioactive polymers onto a titanium or titanium alloy prosthetic metallic material, comprising the following steps:
a) oxidation of the surface of the metallic material, leading to an oxidized surface of the metallic material;
b) grafting a polymer from the oxidized surface of said metallic material, by radical polymerization of a monomer in the presence of the oxidized surface of the metallic material, said radical polymerization comprising an initiation step and a propagation step,
said initiation step being carried out by UV irradiation with a UV source diffusing a power at the surface of the material greater than 72 mW·cm²,
said UV irradiation being carried out for a duration greater than 30 minutes and less than 180 minutes,
said process leading to a titanium or titanium alloy prosthetic metallic material grafted with bioactive polymers.

2. The process according to claim 1, **characterized in that** said initiation step is carried out by UV irradiation with a UV source diffusing a power of between 72 mW·cm² and 20 mW·cm².

3. The process according to claim 1 or 2, **characterized in that** said initiation step is carried out by UV irradiation with a UV source for a duration less than or equal to 90 minutes.

4. The process according to one of claims 2 to 3, **characterized in that** said initiation step is carried out by UV irradiation with a UV source diffusing a power of between 72 mW·cm² and 226 mW·cm².

5. The process according to one of claims 1 to 4, **characterized in that** said initiation step is carried out without heating in addition to UV irradiation.

6. The process according to one of claims 1 to 5, **characterized in that** the concentration of monomer(s) in the solution is between 0.2 and 1 mol·L⁻¹.

7. The process according to one of claims 1 to 6, **characterized in that** the metallic material is a titanium alloy based on nickel, vanadium, aluminium, niobium, and/or molybdenum, in particular an alloy of titanium, aluminium and vanadium, typically the alloy TiAl₆V₄.

8. The process according to one of claims 1 to 7, **characterized in that** said monomer is an olefin.

9. The process according to any one of claims 1 to 8, **characterized in that** the monomer is sodium styrene sulfonate (NaSS).

10. The process according to any one of claims 1 to 8, **characterized in that** the monomer is selected from sulphonates, phosphonates and/or carboxylates, preferably selected from acrylic acid, methacrylic acid, methyl methacrylate (MMA), N-(sodium phenyl sulphonate) acrylamide (NaAS), N-(sodium phenyl sulfonate) methacrylamide (NaMS), ethylene glycol methacrylate, methacrylate phosphate, methacryloyl-di-isopropylidene, vinylbenzylphosphonate (VBP), ethyl 2-[4-(dihydroxyphosphoryl)-2-oxa-butyl]acrylate (MA154), or mixtures thereof.

11. The process according to any one of claims 1 to 10, **characterized in that** said radical polymerization is carried out in the absence of oxygen.

12. The process according to any one of claims 1 to 11, **characterized in that** the initiation step is carried out prior to or concomitantly with the propagation step.

13. The process according to any one of claims 1 to 12, **characterized in that** it comprises a cleaning step carried out prior to the oxidation step, the time between the end of the cleaning step and the start of the oxidation step being less than 16 hours, preferably less than 12 hours.

14. The process according to any one of claims 1 to 13, **characterized in that** the oxidation step a) is carried out by treating the material with an aqueous solution comprising an oxidant and an acid, preferably a mixture of H₂SO₄ and H₂O₂.

15. The process according to any one of claims 1 to 14, **characterized in that** the oxidation step a) is carried out by anodic treatment.

16. The process according to any one of claims 1 to 15, **characterized in that** the grafted metallic material has a grafting rate of said polymer greater than 1.5 µg·cm⁻², preferably greater than 3 µg·cm⁻².
